# EUROPÄISCHE PATENTSCHRIFT

(11) **EP 4 618 950 B1**
(45) Veröffentlichungstag und Bekanntmachung des Hinweises auf die Patenterteilung: **08.04.2026**
(21) Anmeldenummer: 23833053.4
(22) Anmeldetag: 14.12.2023
(51) Int. Cl.: A61K 31/192, A61K 9/00, A61K 47/10, A61K 9/06, A61K 31/164, A61K 31/455, A61K 45/06, A61P 17/00

(54) **STOFFGEMISCH ZUR ANWENDUNG BEI DER TOPISCHEN BEHANDLUNG EINES ONKOLOGISCHEN HAND-FUSS-SYNDROMS, UND VERFAHREN ZUR HERSTELLUNG DES STOFFGEMISCHS**
SUBSTANCE MIXTURE FOR USE IN THE TOPICAL TREATMENT OF AN ONCOLOGICAL HAND-FOOT SYNDROME, AND METHOD FOR PRODUCING THE SUBSTANCE MIXTURE
MÉLANGE DE SUBSTANCES DESTINÉ À ÊTRE UTILISÉ DANS LE TRAITEMENT TOPIQUE D'UN SYNDROME MAIN-PIED ONCOLOGIQUE, ET PROCÉDÉ DE PRODUCTION DU MÉLANGE DE SUBSTANCES

(30) Priorität: 03.01.2023 DE 102023100052
(43) Veröffentlichungstag der Anmeldung: 24.09.2025
(73) Patentinhaber: Klose, Thomas, 56068 Koblenz (DE)
(72) Erfinder: ALTIN, Lena-Maria, 56203 Höhr-Grenzhausen (DE)
(74) Vertreter: Bernsmann, Falk
(86) Internationale Anmeldenummer: PCT/EP2023/085834
(87) Internationale Veröffentlichungsnummer: WO 2024/146763

(56) Entgegenhaltungen:
- WO-A1-2021/204223
- WO-A2-00/62743
- WO-A2-2010/057117
- CA-A1- 2 637 538
- SANTHOSH AKHIL ET AL: "Randomized double-blind, placebo-controlled study of topical diclofenac in the prevention of hand-foot syndrome in patients receiving capecitabine (the D-TORCH study)", TRIALS, vol. 23, no. 1, 1 December 2022 (2022-12-01), GB, XP093137514, ISSN: 1745-6215, Retrieved from the Internet <URL:https://www.ncbi.nlm.nih.gov/pmc/articles/PMC9117836/pdf/13063_2022_Article_6353.pdf> DOI: 10.1186/s13063-022-06353-2

## Beschreibung

### Technisches Gebiet

Die Erfindung betrifft ein Stoffgemisch zur Anwendung bei der dermalen Behandlung einer Hauterkrankung, insbesondere eines onkologischen Hand-Fuß-Syndroms, und ein Verfahren zur Herstellung des Stoffgemischs.

### Stand der Technik

Das onkologische Hand-Fuß-Syndrom (HFS) stellt eine häufig auftretende Nebenwirkung verschiedener tumortherapeutisch eingesetzter Wirkstoffe dar, andere Auslöser dafür sind bislang nicht bekannt.

Typische schmerzhafte Hautreaktion an den Händen und Füßen sind für die Namensgebung des Syndroms verantwortlich. Das National Cancer Institute (NCl) definiert unterschiedliche Schweregrade. Zu Beginn äußert sich das HFS in minimalen Hautveränderungen wie Erythemen oder Schwellungen und Parästhesien (Grad 1). Schmerzen treten zu diesem Zeitpunkt noch keine auf. Blasen, Blutungen und Schwellungen der Haut sowie Schmerzen, die die Patienten in ihrem Alltag beeinträchtigen, sind typisch für ein Grad 2 HFS. Beim dritten und höchsten Schweregrad sind starke Schmerzen und schwere Hautveränderungen verantwortlich für eine Beeinträchtigung der Selbstversorgung. Damit ist das Unvermögen zum Gehen oder zum Greifen umschrieben.

Bezüglich der Ätiologie des HFS werden unterschiedliche Mechanismen diskutiert. Bisher ist der Entstehungsprozess noch nicht abschließend geklärt. Bekannt sind allerdings einige Hypothesen. Darunter fällt die Vermutung eines toxischen Effekts der Arzneistoffe auf Epidermiszellen der Haut. Ein Zusammenhang mit guter Durchblutung wegen des großen Vorkommens an Kapillaren oder mit verstärkter Schweißbildung an Händen und Füßen wird diskutiert.

Bei den auslösenden Wirkstoffen handelt es sich insgesamt um eine inhomogene Gruppe. Es ist folglich noch nicht geklärt, ob das onkologische Hand-Fuß-Syndrom über unterschiedliche Wirkmechanismen ausgelöst wird oder ob es sich um verschiedene Krankheitsbilder handelt, die aber einen ähnlichen Symptomkomplex aufweisen. Allgemein gilt, dass die Inzidenz des Auftretens mit zunehmender Dosis und Therapiedauer steigt. Ebenso führt eine Kombination zweier Stoffe, unter deren Therapie diese Nebenwirkung bekannt ist, zu einem Anstieg der Häufigkeit und Schwere der Reaktion.

Eine Behandlung des HFS ist bislang nur symptomatisch möglich. In Fachkreisen als Standard angesehen wird die 10 % Urea-Creme. Des Weiteren bekannt und gängig ist die Anwendung topischer Glucocorticoide und Schmerzmittel aus der Klasse der nicht-steroidalen-Antirheumatika. Durch diesen Mangel an Behandlungsmöglichkeiten ist der Stellenwert prophylaktischer Maßnahmen umso größer. Zu deren Effektivität gibt es allerdings bisher keine kontrollierten Studien. Patienten müssen geschult werden, während der Therapie die Haut an Händen und Füßen möglichst wenig zu belasten. Dazu sollten Druck, zum Beispiel durch Heben und Tragen von Gegenständen, Reibung, etwa durch enganliegende Kleidung oder Schuhe, und Hitzeeinwirkungen vermieden werden. Die Pflege der Hände und Füße mit kühlenden und feuchtigkeitsspendenden Cremes hat während der Therapie einen besonders wichtigen Stellenwert. Tritt das Syndrom jedoch auf, ist eine Dosisreduktion oder Therapiepause in schweren Fällen nicht vermeidbar, wodurch sich die Behandlung der Krebserkrankung verzögert, gegebenenfalls sogar abgebrochen werden muss und somit deren Erfolg in Frage gestellt sein kann.

Das Dokument WO 2010/057117 A2 offenbart eine Zusammensetzung zur lokalen Anwendung auf der Haut zur Anwendung bei einer Hauterkrankung enthaltend 0,6 Massenprozent Niacin und 4 Massenprozent Ibuprofen, sowie Benzylalkohol als Antioxidans. Das Dokument WO 00/62743 A2 offenbart Zusammensetzungen zur topischen Anwendung bei Hauterkrankungen, enthaltend 3,5 Massenprozent Niacinamid, 1,5 Massenprozent Salizylsäure, 0,5 Massenprozent Vitamin E, sowie EDTA als Antioxidans. Das Dokument CA 2 637 538 A1 offenbart eine Creme zur Behandlung von Akne, enthaltend Ibuprofen, Niacinamid, Ascorbinsäure, Vitamin E. Das Dokument WO 2021/204223 A1 offenbart eine pharmazeutische Zusammensetzung enthaltend Nicotinamid in Gelform zur lokalen Behandlung des onkologischen Fuß-Hand-Syndroms. Gele mit 5 % Massenanteil Nicotinamid sind offenbart. Das Dokument XP093137514 (SANTHOSH AKHIL ET AL: "Randomized double-blind, placebo-controlled study of topical diclofenac in the prevention of hand-foot syndrome in patients receiving capecitabine (the D-TORCH study)", TRIALS, Bd. 23, Nr. 1, 1. Dezember 2022) offenbart die Verwendung einer einprozentigen topischen Diclofenac Zubereitung zur Behandlung des onkologischen Fuß-Hand-Syndroms.

### Technische Aufgabe

Die Aufgabe der Erfindung ist es, ein einfach und sicher anwendbares Medikament zur wirksamen dermalen Behandlung einer Hauterkrankung, insbesondere eines onkologischen Hand-Fuß-Syndroms, zu schaffen.

### Technische Lösung

Die vorliegende Erfindung stellt ein Stoffgemisch gemäß Anspruch 1 bereit, das die technische Aufgabe löst. Vorteilhafte Ausgestaltungen sind Gegenstand der abhängigen Ansprüche.

Das Stoffgemisch ist zur Anwendung bei der topischen Behandlung einer, insbesondere menschlichen, Hauterkrankung vorgesehen. Die Hauterkrankung ist ein onkologisches Hand-Fuß-Syndrom.

Das Stoffgemisch enthält den nicht-steroidalen entzündungshemmenden Wirkstoff Ibuprofen mit einem Massenanteil an dem Stoffgemisch von 3 % bis 6 %.

Das Stoffgemisch enthält Nicotinamid mit einem Massenanteil an dem Stoffgemisch von 2 % bis 6 %.

Das Stoffgemisch enthält vorzugsweise zumindest einen antioxidativen Wirkstoff mit einem Massenanteil an dem Stoffgemisch von 0,01 % bis 10 %.

### Vorteilhafte Wirkungen

Die Haut weist, insbesondere an Händen und Füßen, ein großes Kapillarnetzwerk auf. Dadurch entsteht ein hoher Blutfluss und im Blut zirkulierende Medikamente und deren Abbauprodukte treten in hoher Konzentration auf, wodurch Entzündungen verursacht werden können. Ferner enthält die Haut eine hohe Konzentration von Keratinozyten, die durch ihre hohe Teilungsrate für die meisten Krebsmedikamente besonders anfällig sind.

Nicht-steroidale entzündungshemmende Wirkstoffe (nonsteroidal antiinflammatory drugs - NSAID) werden bei leichten bis mittleren Schmerzen sowie Entzündungsreaktionen eingesetzt und finden sich in vielen apothekenpflichtigen Präparaten zur Anwendung auf der Haut. Diese Wirkstoffe können daher mit der Hauterkrankung verbundene Schmerzen und Entzündungen wirksam lindern und sind dabei sicher und ohne systemische Nebenwirkungen anwendbar.

Nicotinamid ist ein im menschlichen Körper endogen vorkommender Stoff. Es gibt Untersuchungen zum Einsatz von Nicotinamid zur Therapie von Akne oder polymorpher Lichtdermatose. In beiden Fällen handelt es sich um Hauterkrankungen. Bei einer topischen Anwendung werden Nicotinamid antiinflammatorische und antibakterielle Wirkungen zugeschrieben. Des Weiteren gilt Nicotinamid als photoprotektiv sowie juckreizstillend. In Kosmetika ist Nicotinamid ein häufiger Zusatz, um trockener und unreiner Haut entgegenzuwirken. Somit kann Nicotinamid eine Entzündung oder Infektion im Rahmen der Hauterkrankung lindern und durch Juckreiz verursachte zusätzliche Verletzungen der Haut verhindern. Besonders vorteilhaft kann eine Entzündung der Haut durch die Kombination eines nicht-steroidalen entzündungshemmenden Wirkstoffs mit Nicotinamid bekämpft werden, weil die beiden Stoffe die Entzündung durch voneinander unterschiedliche Wirkmechanismen auf komplementäre Weise bekämpfen.

Oxidativer Stress ist eine mögliche Ursache für Hauterkrankungen, verursacht beispielsweise durch Medikamente oder deren Abbauprodukte, die durch die Schweißdrüsen der Haut ausgeschieden werden und bei Kontakt mit Luftsauerstoff Radikale und/oder reaktive Sauerstoffspezies bilden, die eine entzündliche Schädigung auslösen können. Der antioxidative Wirkstoff kann dieser Ursache einer Entzündungsreaktion entgegenwirken und somit die Entzündung auf eine zu dem nicht-steroidalen entzündungshemmenden Wirkstoff und zu Nicotinamid komplementäre Weise bekämpfen.

### Beschreibung der Ausführungsarten

Der zumindest eine nicht-steroidale entzündungshemmende Wirkstoff ist Ibuprofen. Der topische Einsatz von Ibuprofen ist mittels placebokontrollierter Studien besonders gut erforscht. Der Wirkstoff akkumuliert durch seine saure Eigenschaft im entzündeten Gewebe, wodurch das antiinflammatorische Einsatzgebiet zu erklären ist, und verursacht nur geringe lokale Nebenwirkungen.

Das Ibuprofen ist mit einem Massenanteil an dem Stoffgemisch von 3 % bis 6 %, bevorzugt 5 %, in dem Stoffgemisch enthalten. Die genannten Massenanteile führen zu einer sicheren und wirksamen Behandlung der Hauterkrankung.

Das Nicotinamid ist mit einem Massenanteil an dem Stoffgemisch von 2 % bis 6 %, bevorzugt 4 %, in dem Stoffgemisch enthalten. Die genannten Massenanteile führen zu einer sicheren und wirksamen Behandlung der Hauterkrankung.

Das Stoffgemisch enthält vorzugsweise Dexpanthenol mit einem Massenanteil an dem Stoffgemisch von 1 % bis 10 %, bevorzugt von 3 % bis 7 %, besonders bevorzugt 5 %. Bei Dexpanthenol handelt es sich um den Alkohol des Vitamins B5, der Pantothensäure. Im menschlichen Körper wird Dexpanthenol in Pantothensäure umgewandelt. Pantothensäure ist ein Bestandteil des Coenzyms A, welches eine zentrale Funktion in vielen Stoffwechselvorgängen einnimmt. Der Wirkstoff fördert die Epithelisierung und führt so zu einer beschleunigten Wundheilung. Außerdem spendet Dexpanthenol Feuchtigkeit und kühlt. Es ist auch ein juckreizlindernder und entzündungshemmender Effekt bekannt. Folglich handelt es sich um einen wertvollen Bestandteil des Stoffgemischs. Die genannten Massenanteile führen zu einer sicheren und wirksamen Behandlung der Hauterkrankung. Da Dexpanthenol aufgrund anderer Wirkmechanismen die auch von Nicotinamid verursachte entzündungshemmende und juckreizlindernde Wirkung zeigt, ergänzen sich diese beiden Stoffe gut miteinander, um die Hauterkrankung umfassend zu behandeln.

Der zumindest eine antioxidative Wirkstoff umfasst oder ist vorzugsweise Vitamin E, insbesondere Tocopherol. Das Tocopherol ist vorzugsweise synthetisch hergestellt und/oder enthält die acht Stereoisomere des Tocopherols (all-rac-Tocopherol). Eine synthetische Herstellung garantiert eine konstante Produktqualität. Die Verwendung aller Stereoisomere erspart eine aufwändige Trennung der Stereoisomere und vergünstig dadurch die Herstellung des Stoffgemischs.

Das Tocopherol ist vorzugsweise mit einem Massenanteil an dem Stoffgemisch von 0,05 % bis 0,5 %, bevorzugt von 0,1 % bis 0,4 %, besonders bevorzugt 0,2 %, in dem Stoffgemisch enthalten.

Tocopherol besitzt antioxidative Eigenschaften und wird als Zusatz mit einem Massenanteil von 0,2 % auch zur Konservierung lipophiler Arzneimittel eingesetzt. Höhere Konzentrationen führen zu prooxidativer Wirkung und sind daher kontraproduktiv. Neuere Forschungsarbeiten bestätigen einen photoprotektiven Effekt von Vitamin E. Aufgrund der genannten Wirkungen eignet sich Tocopherol mit den genannten Massenanteilen besonders gut für die erfindungsgemäße Anwendung des Stoffgemischs. Besonders vorteilhaft unterstützt Tocopherol die photoprotektive Wirkung des Nicotinamids, sodass diese beiden Stoffe gemeinsam photoinduzierte Schädigungen der Haut verhindern.

Der zumindest eine antioxidative Wirkstoff umfasst oder ist beispielsweise Ascorbinsäure, bevorzugt mit einem Massenanteil an dem Stoffgemisch von 0,01 % bis 1 %, besonders bevorzugt von 0,02 % bis 0,5 %. Die genannten Massenanteile führen zu einer sicheren und wirksamen Behandlung der Hauterkrankung.

Nachteilig an Ascorbinsäure ist, dass die antioxidative Wirkung der Ascorbinsäure vom pH-Wert abhängig ist, der von den weiteren in dem Stoffgemisch enthaltenen Stoffen abhängig ist und am Anwendungsort des Stoffgemischs an der Hautoberfläche unterschiedlich hoch sein kann. Der zumindest eine antioxidative Wirkstoff kann Tocopherol und Ascorbinsäure umfassen.

Das Stoffgemisch enthält beispielsweise Menthol, bevorzugt mit einem Massenanteil an dem Stoffgemisch von 0,1 % bis 10 %, besonders bevorzugt von 0,25 % bis 5 %. Menthol wirkt antiinflammatorisch, kühlend sowie sekundär schmerz- und juckreizstillend und trägt somit vorteilhaft zur Behandlung der Hauterkrankung bei. Außerdem riecht Menthol angenehm, wodurch die Akzeptanz bei Patientinnen und Patienten erhöht wird.

Der zumindest eine antioxidative Wirkstoff umfasst oder ist beispielsweise **N-**Acetylcystein (NAC), bevorzugt mit einem Massenanteil an dem Stoffgemisch von 5 % bis 10 %. Nachteilig an N-Acetylcystein sind die möglichen Nebenwirkungen in Form von Irritationen und ein störender Geruch.

Das Stoffgemisch enthält beispielsweise Propolis. Propolis umfasst eine Vielzahl wirksamer Bestandteile (circa 150 identifiziert), unter anderem Flavonoide, phenylsubstituierter Carbonsäuren und ätherische Öle. Propolis wirkt adstringierend, antimikrobiell, antiphlogistisch, immunstimulierend und zytostatisch und kann somit in vielfältiger Weise zur Behandlung der Hauterkrankung beitragen. Allerdings hat Propolis als Naturprodukt eine variable Zusammensetzung, sodass eine gleichbleibende Produktqualität schwierig zu gewährleisten ist und ein allergenes Risiko bei topischer Anwendung besteht. Ferner erschwert die harzige Form von Propolis dessen homogene Verarbeitung in dem Stoffgemisch.

Das Stoffgemisch enthält vorzugsweise eine Trägersubstanz, wobei die Trägersubstanz ein Schaum, ein Gel, eine Creme oder eine Salbe umfasst oder ist. Aufgrund der Schmerzempfindlichkeit der behandelten Haut eignet sich eine leicht aufzutragende, schnell einziehende und/oder kühlende Trägersubstanz besonders gut. Unter diesen Gesichtspunkten ist ein Schaum besonders bevorzugt.

Die Trägersubstanz umfasst oder ist vorzugsweise eine nichtionische und/oder hydrophile Creme. Eine Creme, insbesondere eine hydrophile Creme zeichnet sich dadurch aus, dass sie leicht aufzutragen ist und schnell einzieht. Eine nichtionische Creme zeigt keine ionischen Wechselwirkungen mit den in dem Stoffgemisch enthaltenen Wirkstoffen, die die Wirksamkeit der Wirkstoffe beeinträchtigen könnten.

Die Trägersubstanz enthält vorzugsweise Wasser mit einem Massenanteil an der Trägersubstanz von 40 % bis 80 %, bevorzugt von 60 % bis 70 %. Ein hoher Wasseranteil der Trägersubstanz bewirkt eine leichte Textur des Stoffgemischs, sodass dieses leicht aufzutragen ist und schnell einzieht. Außerdem kühlt das Wasser die Haut, wenn es verdunstet. Wasser wirkt außerdem als natürlicher Penetrationsbeschleuniger und führt so zu einem verbesserten transdermalen Wirkstofftransport.

Ein erfindungsgemäßes Verfahren dient zur Herstellung des erfindungsgemäßen Stoffgemischs zur erfindungsgemäßen Anwendung. Das Verfahren umfasst ein Vermischen der Stoffe des Stoffgemischs, wobei das Stoffgemisch während des Vermischens vorzugsweise auf eine Temperatur von 40 °C bis 90 °C, insbesondere von 50 °C bis 80 °C, besonders bevorzugt auf 60 °C bis 70 °C, erwärmt wird. Durch das Erwärmen, das beispielsweise in einem Wasserbad erfolgt, wird sichergestellt, dass sich die Stoffe gleichmäßig vermischen, sodass ein homogenes Stoffgemisch entsteht. Ein homogenes Stoffgemisch sorgt für eine gut reproduzierbare Wirkung bei der Anwendung des Stoffgemischs. Das Stoffgemisch darf nicht zu stark erwärmt werden, damit die darin enthaltenen Stoffe nicht thermisch in ihrer Wirksamkeit beeinträchtigt werden. Daher ist ein Erwärmen auf die genannten Temperaturbereiche für eine hohe und gut reproduzierbare Wirksamkeit des Stoffgemischs optimal.

### Beispiele

Das Stoffgemisch besteht beispielsweise aus folgenden Stoffen mit den genannten Massenanteilen an dem Stoffgemisch:

| | |
|---|---|
| Ibuprofen | 5 % |
| Nicotinamid | 4 % |
| Dexpanthenol | 5 % |
| all-rac-Tocopherol | 0,2 % |
| Trägersubstanz | ad 100 % |

Die Trägersubstanz ist beispielsweise die nichtionische hydrophile Creme SR "neu" (Unguentum emulsificans nonionicum aquosum) gemäß dem Neuen Rezeptur-Formularium (NRF). Diese Creme besteht aus folgenden Stoffen mit den angegebenen Massenanteilen an der Creme:

| | |
|---|---|
| Cetylstearylalkohol | 16,8 % |
| Macrogol-20-cetylstearylether | 4,2 % |
| 2-Ethylhexyllaurat | 10,0 % |
| Glycerol 85 % | 5,0 % |
| Kaliumsorbat | 0,14 % |
| wasserfreie Citronensäure | 0,07 % |
| gereinigtes Wasser | ad 100 % |

Der hydrophile Anteil der nichtionische hydrophile Creme SR "neu" liegt bei 70 %. Glycerol 85 % ist als nicht flüchtige, hydrophile Komponente mit fünf Massenprozent enthalten. Dieses trägt als Feuchthaltemittel, auch Moisturizer genannt, zur Konsistenz und pflegenden Eigenschaft der Trägersubstanz bei. Es begünstigt die Regeneration der Hornschicht der Haut. Der hohe wässrige Anteil führt zu mikrobieller Anfälligkeit und erfordert somit eine Konservierung der Creme. Dazu ist Kaliumsorbat und wasserfreie Citronensäure im Verhältnis zwei zu eins zugesetzt. Hierbei handelt es sich um als gesundheitlich unproblematisch eingestufte, gut verträgliche Stoffe. Durch die Konservierung resultiert letztlich ein pH-Wert von etwa 4,8. Als lipophiler Bestandteil ist 2-Ethylhexyllaurat zu zehn Prozent enthalten. Es handelt sich chemisch gesehen um einen Carbonsäureester. Diesem Stoff ist es zuzuschreiben, dass die Trägersubstanz schnell in die Haut einzieht. Als weiterer strukturgebender Bestandteil sind Emulgatoren zugesetzt. Die "neue" Variante der nichtionischen hydrophilen Creme setzt Cetylstearylalkohol, einen Fettalkohol, und Macrogol-20-cetylstearylether, einen hydrophilen ethoxylierten Cetylstearylalkohol, als Emulgatoren ein. Die Mischung wird als nichtionischer emulgierender Cetylstearylalkohol, "Cetomacrogolwachs BP" bezeichnet. Dabei handelt es sich um einen selbstemulgierenden Komplexemulgator. Cetylstearylalkohole bergen ein gewisses allergenes Potential, was jedoch hauptsächlich bei okklusiver Anwendung auftritt.

Die nichtionische hydrophile Creme SR "neu" bewirkt einen geringen okklusiven Effekt, woraus ein gute Akzeptanz bei Patientinnen und Patienten resultiert. Die Creme hat eine nur wenig starke Tiefenwirkung, was allerdings wegen des Wirkorts unproblematisch ist, da das Stoffgemisch nur in den oberen Hautschichten wirken muss. Dadurch reichert sich der nicht-steroidale entzündungshemmende Wirkstoff, insbesondere Ibuprofen, vorteilhafterweise im Stratum corneum an.

Die Stabilität des beispielhaften Stoffgemischs mit der nichtionischen hydrophilen Creme SR "neu" als Trägersubstanz wurde in einer Stabilitätsuntersuchung über sechs Monate erprobt. Anhand dieser wurde bestätigt, dass das Stoffgemisch kühl gelagert über mindestens zwei Monate stabil bleibt.

Für die Stabilitätsuntersuchung wurde das Stoffgemisch viermal hergestellt. Der Unterschied war das Primärpackmittel. Je zweimal wurde das Stoffgemisch in Aluminiumtuben und zweimal in Kruken abgefüllt. Der direkte Vergleich fand bei gleichen Zusammensetzungen und Primärpackmitteln, aber bei verschiedenen Lagertemperaturen (Kühlschranktemperatur von 2 °C bis 8 °C oder Raumtemperatur) statt. Der Beobachtungszeitraum erstreckte sich über 6 Monate. Die Dokumentationspunkte starteten wöchentlich im ersten Monat, verlängerten sich dann auf zweiwöchentlich im zweiten und dritten Monat bis hin zu monatlich.

Untersucht wurde die Homogenität. Ein homogener Cremestrang sieht überall gleich aus und zeigt kein austretendes Wasser. Es wurde auch geprüft, ob Kristalle spürbar sind und ob die Viskosität gleichbleibend war. Viskositätszunahmen werden deutlich durch rissigere, festere oder schneller abreißende Salben-stränge und geben Hinweise auf lavierte Inkompatibilitäten. Ein frischer Salbenstrang der Cremes wurde jeweils optisch verglichen und fotografisch festgehalten sowie haptisch auf spürbare Kristalle geprüft.

In Vorversuchen hat sich eine gute Verträglichkeit des beispielhaften Stoffgemischs mit der nichtionischen hydrophilen Creme SR "neu" als Trägersubstanz auf gesunder Haut gezeigt.

Eine weitere beispielhafte Trägersubstanz ist die Kühlcreme (Unguentum leniensis) gemäß dem Deutschen Arzneibuch. Diese Kühlcreme besteht aus folgenden Stoffen mit den angegebenen Massenanteilen:

| | |
|---|---|
| Gelbes Wachs | 7 % |
| Cetylpalmitat | 8 % |
| raffiniertes Erdnussöl | 60 % |
| gereinigtes Wasser | 25 % |

Die Kühlcreme bildet eine lipophile Trägersubstanz und wird bei Pruritus, Xerosis und subakuten Dermatiden eingesetzt. Sie enthält keinen Emulgator, wodurch die Kühlcreme sehr gut verträglich aber wenig stabil ist. Da das enthaltene Wasser nur mechanisch eingeschlossen wird, kann es leicht zu einer Phasentrennung kommen. Wenn es beim Auftragen zur Phasentrennung kommt, wird Wasser frei und verdunstet, was zu einer vorteilhaften Kühlung der Haut führt. Die geringe Stabilität erschwert die Einarbeitung von Wirkstoffen in die Trägersubstanz. Da die Kühlcreme nur sehr langsam einzieht und stark fettet, ist die Akzeptanz bei Patientinnen und Patienten gering.

Eine weitere beispielhafte Trägersubstanz ist die hydrophobe Basiscreme gemäß dem Deutschen Arzneimittel-Codex (DAC). Diese hydrophobe Basiscreme besteht aus folgenden Stoffen mit den angegebenen Massenanteilen:

| | |
|---|---|
| Triglyceroldiisostearat | 3,0 % |
| Isopropylpalmitat | 2,4 % |
| Hydrophobes Basisgel DAC | 24,6 % |
| Kaliumsorbat | 0,14 % |
| wasserfreie Citronensäure | 0,07 % |
| Magnesiumsulfat-Heptahydrat | 0,5 % |
| Glycerol 85 % | 5,0 % |
| gereinigtes Wasser | ad 100 % |

Die hydrophobe Basiscreme ist vorteilhafterweise zur Einarbeitung der übrigen Stoffe des Stoffgemischs geeignet, hydratisierend und wenig okklusiv. Nachteilig für die Akzeptanz bei Patientinnen und Patienten sind jedoch die schwere Streichfähigkeit und der stark fettende Charakter der hydrophoben Basiscreme.

Die Streichfähigkeit der hydrophoben Basiscreme kann beispielsweise durch einen Zusatz von 10 % Glycerol 85 % und optional 15 % raffiniertem Nachtkerzenöl verbessert werden. Dies führt allerdings zu einer sehr klebrigen Konsistenz.

In einer Voruntersuchung wurde ein erfindungsgemäßes Stoffgemisch in Form einer Creme bestehend aus Ibuprofen mit einem Massenanteil von 5 % an dem Stoffgemisch, Nicotinamid mit einem Massenanteil von 4 % an dem Stoffgemisch und der nichtionischen hydrophilen Creme SR "neu" an 13 Patienten erprobt. Am Anfang der Voruntersuchung zeigten die Patienten ein onkologisches Hand-Fuß-Syndrom in unterschiedlich starker Ausprägung. Alle Patienten erhielten vor und während der Voruntersuchung eine medikamentöse Tumortherapie mit unterschiedlichen Medikamenten. Die Patienten haben die Creme während eines Zeitraums von 14 Tagen zweimal täglich auf Hände und Füße aufgetragen.

Die folgende Tabelle gibt einen Überblick über die Voruntersuchung:

| Patient Nr. | Tumor-Medikamente | Ausgangssituation | Ergebnis |
|---|---|---|---|
| 1 | Irinotecan + Bevacizumab + Panitumumab | starke Beschwerden und Einschränkungen im Alltag | stetige Besserung; ab Tag 9 symptomfrei |
| 2 | nab-Paclitaxel + Carboplatin + Pertuzumab + Trastuzumab | sehr starke Beschwerden und Einschränkungen im Alltag | sofortige Wirkung gegen Juckreiz und Brennen; stetige Besserung; an Tag 14 symptomfrei |
| 3 | Capecitabin | Haut an Daumen aufgeplatzt; wenige Einschränkungen im Alltag | subjektiv deutliche Besserung; ein Daumen an Tag 5 verheilt; |
| 4 | Capecitabin | Haut an Fingern aufgeplatzt; mäßige Einschränkungen im Alltag | subjektiv deutliche Besserung; objektiv keine Änderung der Symptome erkennbar |
| 5 | Capecitabin | rissige, gereizte Nagelhaut an Händen und raue Haut an den Füßen; wenige Einschränkungen im Alltag | ab Tag 11 symptomfrei |
| 6 | Capecitabin | Blasen, Rötung, Schmerzen; verhärtete, schmerzende Fingerkuppen mit sich abschälender Haut; Beschwerden an Füßen, sodass laufen erschwert war; Füße im Bereich unter den Zehen und an der Ferse sehr stark gerötet und verhärtet; entzündete Zehennägel | Füße und Hände schnell geschmeidiger; Rötungen schnell zurückgegangen; Auftreten und Laufen schnell schmerzfrei; keine Schmerzen mehr trotz weiterer Hautabschälung; keine erneute Entzündung |
| 7 | Capecitabin | minimale Rötung, schmerzempfindlich, kälteempfindlich an Händen und Füßen | keine Besserung, starkes Kribbeln an Füßen an Tag 5, Abbruch an Tag 6 |
| 8 | liposomales Irinotecan | schmerzhafte Rillen an Fingern | ab Tag 5 starkes Kribbeln an Füßen, leichte Besserung an Tag 13 |
| 9 | Capecitabin | Hautrötung | kein erkennbarer Effekt |
| 10 | Capecitabin | trockene Haut, Schmerzen, Hautablösungen an Füßen | deutliche Besserung nach 12 und 14 Tagen |
| 11 | Docetaxel | Hände: Schmerzen, Blasen, Hautablösungen, Fingernägel eingerissen | nach 1 Woche keine Hautabschälung mehr; nach 2 Wochen keine Blasen mehr, Schmerzen nur noch den Fingerspitzen; Beschwerden und Einschränkungen insgesamt schwankend zwischen mäßig und stark |
| 12 | Trastuzumab, Pertuzumab | Rötungen an Händen und Armen | nach 2 Wochen symptomfrei |
| 13 | Docetaxel, Epirubicin | Füße: Schmerzen, trockene Haut, Verdickungen an Ferse und Zehen | bis Tag 7 mäßige Einschränkungen; danach nur noch wenige Einschränkungen |

Zusammenfassend zeigt die Voruntersuchung bei 10 der 13 Patienten zumindest subjektiv eine Linderung der Symptome des onkologischen Hand-Fuß-Syndroms durch die Anwendung eines erfindungsgemäßen Stoffgemischs in Form der untersuchten Creme. Bei zwei Patienten zeigte die Anwendung keine erkennbare Wirkung. Nur bei einem Patienten führte die Anwendung zu einer Verschlechterung des Befindens. Die Voruntersuchung stützt somit die positive Wirkung eines erfindungsgemäßen Stoffgemischs zur Anwendung bei der dermalen Behandlung einer Hauterkrankung, insbesondere eines onkologischen Hand-Fuß-Syndroms.

## Patentansprüche

1. Stoffgemisch zur Anwendung bei der topischen Behandlung eines onkologischen Hand-Fuß-Syndroms,
a. wobei das Stoffgemisch Nicotinamid mit einem Massenanteil an dem Stoffgemisch von 2 % bis 6 % enthält,
**dadurch gekennzeichnet, dass**
b. das Stoffgemisch Ibuprofen mit einem Massenanteil an dem Stoffgemisch von 3 % bis 6 % enthält.

2. Stoffgemisch zur Anwendung gemäß Anspruch 1,
wobei das Ibuprofen mit einem Massenanteil an dem Stoffgemisch von 5 % in dem Stoffgemisch enthalten ist.

3. Stoffgemisch zur Anwendung gemäß Anspruch 1,
wobei das Nicotinamid mit einem Massenanteil an dem Stoffgemisch von 4 % in dem Stoffgemisch enthalten ist.

4. Stoffgemisch zur Anwendung gemäß Anspruch 1,
wobei das Stoffgemisch Dexpanthenol mit einem Massenanteil an dem Stoffgemisch von 1 % bis 10 % enthält.

5. Stoffgemisch zur Anwendung gemäß Anspruch 1
wobei das Stoffgemisch einen antioxidativen Wirkstoff mit einem Massenanteil an dem Stoffgemisch von 0,01 % bis 10 % umfasst.

6. Stoffgemisch zur Anwendung gemäß Anspruch 5,
wobei der antioxidative Wirkstoff Vitamin E umfasst.

7. Stoffgemisch zur Anwendung gemäß Anspruch 6,
wobei das Vitamin E in Form von Tocopherol mit einem Massenanteil an dem Stoffgemisch von 0,05 % bis 0,5 % in dem Stoffgemisch enthalten ist.

8. Stoffgemisch zur Anwendung gemäß Anspruch 5,
wobei der antioxidative Wirkstoff Ascorbinsäure mit einem Massenanteil an dem Stoffgemisch von 0,01 % bis 1 % umfasst.

9. Stoffgemisch zur Anwendung gemäß Anspruch 1,
wobei das Stoffgemisch Menthol mit einem Massenanteil an dem Stoffgemisch von 0,1 % bis 10 % enthält.

10. Stoffgemisch zur Anwendung gemäß Anspruch 1,
wobei das Stoffgemisch eine Trägersubstanz enthält, wobei die Trägersubstanz ein Schaum, ein Gel, eine Creme oder eine Salbe umfasst.

11. Stoffgemisch zur Anwendung gemäß Anspruch 1,
wobei das Stoffgemisch eine Trägersubstanz enthält, wobei die Trägersubstanz eine nichtionische und/oder hydrophile Creme umfasst.

12. Stoffgemisch zur Anwendung gemäß Anspruch 1,
wobei das Stoffgemisch eine Trägersubstanz enthält, wobei die Trägersubstanz Wasser mit einem Massenanteil an der Trägersubstanz von 40 % bis 80 % enthält.

13. Verfahren zur Herstellung eines Stoffgemischs gemäß einem der Ansprüche 1 bis 12 zur Anwendung bei der topischen Behandlung eines onkologischen Hand-Fuß-Syndroms,
umfassend ein Vermischen der Stoffe des Stoffgemischs, wobei das Stoffgemisch während des Vermischens auf eine Temperatur von 40 °C bis 90 °C erwärmt wird.

## Claims

1. A mixture of substances for use in the topical treatment of an oncological hand-foot syndrome,
a. wherein the mixture of substances contains nicotinamide in a mass fraction of 2 % to 6 % of the mixture of substances,
**characterised in that**
b. the mixture of substances contains ibuprofen in a mass fraction of 3 % to 6 % of the mixture of substances.

2. The mixture of substances for use according to claim 1,
wherein the ibuprofen is contained in the mixture of substances in a mass fraction of 5 % of the mixture of substances.

3. The mixture of substances for use according to claim 1,
wherein the nicotinamide is contained in the mixture of substances in a mass fraction of 4 % of the mixture of substances.

4. The mixture of substances for use according to claim 1,
wherein the mixture of substances contains dexpanthenol in a mass fraction of 1 % to 10 % of the mixture of substances.

5. The mixture of substances for use according to claim 1
wherein the mixture of substances comprises an antioxidant agent in a mass fraction of 0.01 % to 10 % of the mixture of substances.

6. The mixture of substances for use according to claim 5,
wherein the antioxidant agent comprises vitamin E.

7. The mixture of substances for use according to claim 6,
wherein the vitamin E is contained in the mixture of substances in the form of tocopherol in a mass fraction of 0.05 % to 0.5 % of the mixture of substances.

8. The mixture of substances for use according to claim 5,
wherein the antioxidant agent comprises ascorbic acid in a mass fraction of 0.01 % to 1 %.of the mixture of substances.

9. The mixture of substances for use according to claim 1,
wherein the mixture of substances contains menthol in a mass fraction of 0.1 % to 10 % of the mixture of substances.

10. The mixture of substances for use according to claim 1,
wherein the mixture of substances contains a carrier substance, wherein the carrier substance comprises a foam, a gel, a cream or an ointment.

11. The mixture of substances for use according to claim 1,
wherein the mixture of substances contains a carrier substance, wherein the carrier substance comprises a non-ionic and/or hydrophilic cream.

12. The mixture of substances for use according to claim 1,
wherein the mixture of substances contains a carrier substance, wherein the carrier substance contains water in a mass fraction of 40 % to 80 % of the carrier substance.

13. A method for producing a mixture of substances according to one of claims 1 to 12 for use in the topical treatment of an oncological hand-foot syndrome, the method comprising mixing the substances of the mixture of substances, wherein the mixture of substances is heated to a temperature of 40 °C to 90 °C during mixing.

## Revendications

1. Mélange de substances destiné à être utilisé dans le traitement topique d'un syndrome oncologique main-pied,
a. le mélange de substances contenant du nicotinamide dans une proportion massique comprise entre 2 % et 6 % du mélange de substances,
**caractérisé en ce que**
b. le mélange de substances contient de l'ibuprofène dans une proportion massique comprise entre 3 % et 6 % du mélange de substances.

2. Mélange de substances destiné à être utilisé selon la revendication 1, l'ibuprofène étant contenu dans le mélange de substances dans une proportion massique de 5 % dans le mélange de substances.

3. Mélange de substances destiné à être utilisé selon la revendication 1,
le nicotinamide étant contenu dans le mélange de substances dans une proportion massique de 4 % dans le mélange de substances.

4. Mélange de substances destiné à être utilisé selon la revendication 1,
le mélange de substances comprenant du dexpanthénol dans une proportion massique comprise entre 1 % et 10 % du mélange de substances.

5. Mélange de substances destiné à être utilisé selon la revendication 1 le mélange de substances comprenant un agent antioxydant dans une proportion massique comprise entre 0,01 % et 10 % du mélange de substances.

6. Mélange de substances destiné à être utilisé selon la revendication 5, l'agent antioxydant comprenant de la vitamine E.

7. Mélange de substances destiné à être utilisé selon la revendication 6,
la vitamine E étant contenue dans le mélange de substances sous forme de tocophérol dans une proportion massique comprise entre 0,05 % et 0,5 % du mélange de substances.

8. Mélange de substances destiné à être utilisé selon la revendication 5, l'agent antioxydant comprenant de l'acide ascorbique dans une proportion massique comprise entre 0,01 % et 1 % du mélange de substances.

9. Mélange de substances destiné à être utilisé selon la revendication 1, le mélange de substances contenant du menthol dans une proportion massique comprise entre 0,1 % et 10 % du mélange de substances.

10. Mélange de substances destiné à être utilisé selon la revendication 1, le mélange de substances contenant une substance support, la substance support comprenant une mousse, un gel, une crème ou une pommade.

11. Mélange de substances destiné à être utilisé selon la revendication 1, le mélange de substances contenant une substance support, la substance support comprenant une crème non ionique et/ou hydrophile.

12. Mélange de substances destiné à être utilisé selon la revendication 1, le mélange de substances contenant une substance support, la substance support contenant de l'eau dans une proportion massique de 40 % à 80 % de la substance support.

13. Procédé de fabrication d'un mélange de substances selon l'une des revendications 1 à 12 destiné à être utilisé dans le traitement topique d'un syndrome oncologique main-pied,
le procédé comprenant une étape de mélanger les substances du mélange de substances, le mélange de substances étant chauffé pendant l'étape de mélanger à une température comprise entre 40 °C et 90 °C.
